# EUROPEAN PATENT SPECIFICATION

(11) **EP 0 458 442 B1**
(45) Date of publication and mention of the grant of the patent: **20.08.1997**
(21) Application number: 91301818.0
(22) Date of filing: 05.03.1991
(51) Int. Cl.: C07D 251/50, C09B 62/51

(54) **Colorants**
Farbstoffe
Colorants

(30) Priority: 22.05.1990 GB 9011452
(43) Date of publication of application: 27.11.1991
(73) Proprietor: BASF AKTIENGESELLSCHAFT, 67056 Ludwigshafen (DE)
(72) Inventor: Anderton, Kenneth, Bury, Lancashire (GB)
(74) Representative: Kiddle, Simon John

(56) References cited:
- EP-A- 0 111 288
- US-A- 4 649 193

## Description

This invention relates to coloured compounds having cellulose reactive groups, to a process for the coloration of materials having -OH and/or -NH- groups, and to materials having -OH and -NH- groups when coloured by the coloured compound.

According to the present invention there is provided a coloured compound which, in the free acid form, is of Formula (1): 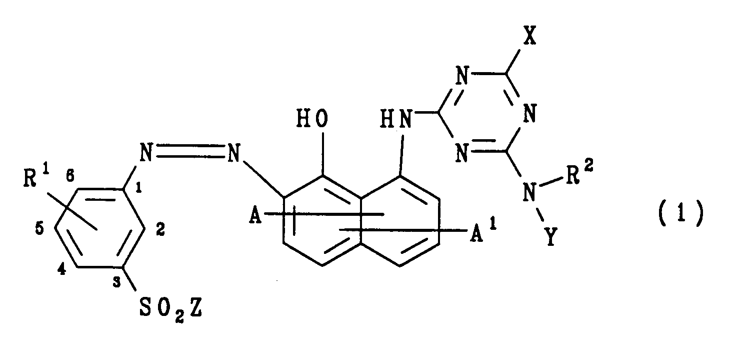 wherein:
- R¹: is H, alkyl, alkoxy, CO₂H or SO₃H;
- Z: is -CH=CH₂, or a group which yields -CH=CH₂ on treatment with aqueous alkali;
- A and A¹: are each independently H or SO₃H provided at least one of A and A¹ is SO₃H;
- X: is Cl or F;
- R²: is H, alkyl or alkenyl; and
- Y: is phenyl substituted by one or two groups selected from CO₂H and SO₃H.

R¹ is preferably H, C₁₋₄-alkyl, C₁₋₄-alkoxy, CO₂H or SO₃H, more preferably H, methyl, methoxy, CO₂H or SO₃H, and especially methoxy or H. When R¹ is C₁₋₄-alkyl, C₁₋₄-alkoxy, CO₂H or SO₃H it is preferably at the 4 or more preferably the 6 position relative to the diazo group.

R² is preferably H, C₁₋₄-alkyl or C₃₋₄-alkenyl, more preferably H, methyl, ethyl, iso-propyl or allyl. It is particularly preferred that R² is H.

Both A and A¹ are preferably SO₃H. When A is SO₃H it is preferably at the 3 position relative to the OH group connected to the same naphthalene ring system. When A¹ is SO₃H it is preferably at the 5 or 6 position, especially the 6 position, relative to the OH group connected to the same naphthalene ring system. It is particularly preferred that both A and A¹ are SO₃H and are respectively at the 3 and 6 positions or 3 and 5 positions, especially the 3 and 6 position, relative to the OH group connected to the same naphthalene ring system.

X is preferably Cl.

Z is preferably -CH=CH₂, -CH₂CH₂SSO₃H or -CH₂CH₂OSO₃H.

Examples of preferred groups represented by Y are, 2, 3 or 4-sulphophenyl; 2,4-disulphophenyl; 2,5-disulphophenyl; 3,5-disulphophenyl; 2-carboxyphenyl; 3-carboxyphenyl; 4-carboxyphenyl; 2,3-dicarboxyphenyl; 2,4-dicarboxyphenyl; 2,5-dicarboxyphenyl; 2,6-dicarboxyphenyl; 3,4-dicarboxyphenyl; 3,5-dicarboxyphenyl; 4-carboxy-2-sulphophenyl; 3-carboxy-5-sulphophenyl; 2-carboxy-4-sulphophenyl; and 2-carboxy-5-sulphophenyl.

Examples of preferred groups represented by R¹ are 6-methoxy, 6-methyl, 4-methoxy, 4-methyl, 6-sulpho and 6-carboxy.

A particularly preferred subclass of compound according to the present invention is a compound which, in the free acid form, has the formula: 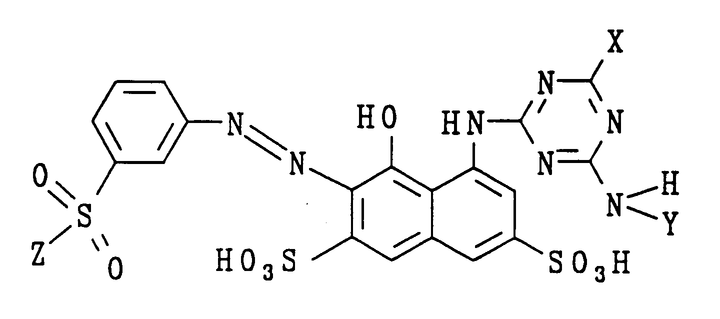 wherein X, Y and Z are as hereinbefore defined.

It will be understood that the present invention relates not only to a compound of Formula (1) in its free acid form but also to a salt thereof, particularly the salt with an alkali metal, ammonia or an amine, especially the sodium or lithium salt or the mixed sodium/lithium salt.

The compound of the present invention is particularly useful for the coloration of cellulosic textiles, for example by pad-batch and pad-roll dyeing processes wherein textile piece goods are impregnated with a liquor containing a coloured compound of Formula (1) and then allowed to react after having been batched or rolled up. The batch is generally rotated slowly while the reactive dye fixes to the textile piece goods.

According to a further aspect of the present invention there is provided a process for the manufacture of a dye of the Formula (1) wherein an amine of Formula (2) is reacted with a diazotising agent, such as HNO₂, in the cold, preferably below 5°C, to give a diazonium salt which is coupled to a compound of Formula (4) in which H is hydrogen, and the resultant azo compound condensed with a compound of formula YNR²H (wherein Y and R² are as defined above), preferably in the presence of base, to give a compound of Formula (1).

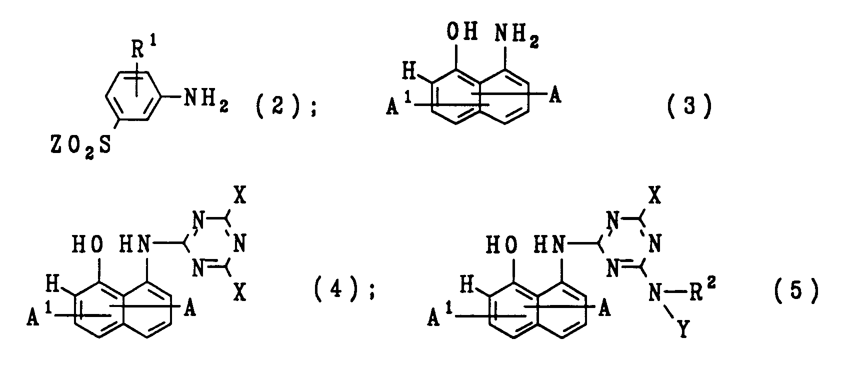

An alternative process for the manufacture of dyes of Formula (1) is to react a cyanuric halide with a compound of formula YNR²H to give a dihalotriazine intermediate which is condensed with an amine of Formula (3) in which H is hydrogen to give a monohalotriazinyl intermediate of Formula (5) in which H is hydrogen, to which is coupled a diazotised amine of Formula (2) using the coupling conditions described above.

A further alternative process for the manufacture of dyes of Formula (1) is the condensation of a compound of Formula (3) with a cyanuric halide to give a dihalotriazinyl naphthalene intermediate which is subsequently reacted with an amine of formula YNR²H to give a monohalotriazinyl intermediate of Formula (5). An amine of Formula (2) is reacted with a diazotising agent such as HNO₂ in the cold, preferably below 5°C, to give a diazonium salt which is coupled to the monohalotriazine intermediate to give a compound of Formula (1).

In Formulae (2), (3), (4) and (5), R¹, R², Z, X, Y, A and A¹ are as hereinbefore defined.

According to a further aspect of the present invention there is provided a process for the colouration materials having -OH and/or -NH- groups by applying thereto a compound of Formula (1).

The -OH group-containing materials include natural or synthetic hydroxyl group-containing materials such as cellulosic textile materials, and regenerated products thereof. Examples of the cellulosic textile materials include cotton and other vegetable fibres such as linen, hemp, jute, flax and ramie fibres. Examples of the regenerated cellulosic textile materials include viscose staple and filament viscose.

The -NH- group-containing materials include synthetic and natural polyamides and polyurethanes. Examples of such materials include animal furs such as wool and silk, leather, nylon-6,6 and nylon-6.

The dyeing may be carried out in any suitable manner depending on the physical and chemical properties of the fibre materials. For example, cellulosic materials can be coloured using the compound of Formula (1) by exhaustion dyeing; padding, including cold pad batch dyeing; or by printing.

The dyeing of cellulosic textile materials and regenerated products thereof is preferably carried out in the presence of an acid binding agent together with a hydrotropic agent, a penetrant or a level dyeing agent. The acid binding agent for promoting the exhaustion may be added to a dye bath before, during or after reaching the desired temperature for dyeing.

Examples of preferred acid binding agents used for fixing the compound of the present invention to the cellulosic textile materials include water-soluble basic salts such as alkali or alkaline earth metals and inorganic or organic acids or compounds capable of liberating alkalis under heating conditions. The particularly preferred acid binding agents are alkali metal hydroxides and alkali metal salts of inorganic or organic acids having a weak or medium strength. Especially preferred are sodium salts and potassium salts. Specific examples of acid binding agents include sodium hydroxide, potassium hydroxide, sodium hydrogencarbonate, sodium carbonate, sodium formate, potassium carbonate, sodium dihydrogenphosphate, disodium hydrogenphosphate, trisodium phosphate, sodium silicate and sodium trichloroacetate.

The padding can be carried out by padding the cellulosic textile materials at ambient temperature or an elevated temperature, and drying, steaming or dry-heating the materials to perform the dye-fixation.

The cold pad batch dyeing can be carried out by padding the cellulosic textile materials with a padding liquor at ambient temperature and allowing them to stand on a frame for 3 hours or more or overnight followed by washing with water and drying.

In a typical pad-batch dyeing 25 parts of a compound of Formula (1) is dissolved in hot water (1000 parts) and cooled to 25°C. Aqueous NaOH solution (e.g. 5.5 parts of 32.5% solution) and 50° Be water glass (ca. 150 parts) is then thoroughly mixed in. The material to be coloured is then padded with the mixture, batched up, wrapped tightly with a polyethylene film and allowed to stand at above 5°C for about 20 hours.

Thereafter, the cloth is washed with water, washed at the boil with a detergent, washed again with water and dried to give dyed cloth.

The printing can be carried out in a one-phase or two-phase manner. The one-phase printing may be conducted by printing the fibre materials with a printing paste containing an acid binding agent such as sodium hydrogencarbonate, sodium carbonate or the like, followed by steaming at a temperature of 100° to 160°C. The two-phase printing may be conducted by printing the fibre materials with a neutral or weakly acidic printing paste, and passing the materials through a hot alkaline bath containing an electrolyte or over-padding the materials with an alkaline padding liquor containing an electrolyte, followed by a steaming or dry-heating treatment.

For the preparation of the printing paste, a paste or emulsifier such as sodium alginate, starch ether or the like may be used, and if desired, together with a conventional auxiliary agent such as urea and/or dispersing agent.

The dyeing of natural or synthetic polyamide and polyurethane fibre materials can be carried out by exhaust dyeing in an acid or weakly acidic bath whilst controlling the pH, and then making the bath neutral, or in some cases alkaline to perform the fixation. The dyeing temperature is preferably in the range of 60° to 120°C, optionally in the presence of a conventional level dyeing agent.

Alternatively, the compound of Formula (1) may be applied to materials having -OH and/or -NH- groups by ink jet printing.

A further aspect of the present invention comprises materials having -OH and/or -NH- groups, and especially cellulosic materials such as cotton, when coloured by a compound of the present invention.

The present invention is further illustrated by the following examples in which all parts and percentages are by weight unless stated otherwise.

### Example 1

### Preparation of the compound of Formula (6) in which R¹ and R² are H and Y is 4-sulpho-2-carboxyphenyl

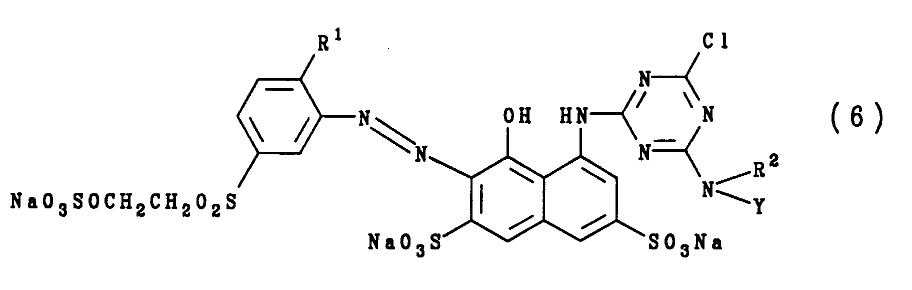

2N NaOH (aqueous) was added to a suspension of 1-amino-8-naphthol-3,6-disulphonic acid (319 parts) in water (5000 parts) until a complete solution of approximately pH 6.0 was obtained. This solution was added slowly to a suspension obtained by gradual addition of a solution of cyanuric chloride (203 parts) in acetone (3950 parts) to water (5000 parts) at 0-5°C. The resultant mixture was stirred for a further 1.5 hours at 0-5°C to give a solution of coupling component.

To a stirred mixture of 3-beta-sulphatoethyl sulphonyl aniline (309 parts) in water (2500 parts) at 0-5°C was added hydrochloric acid (aqueous) (34.5% strength, 708 parts) followed by sodium nitrite solution (2N, 605 parts by volume). The mixture was stirred for 15 minutes at 0-5°C before destroying excess nitrous acid by addition of sulphamic acid. The resultant suspension of diazonium salt was added to a solution of the above coupling component, the pH was raised to between 6.0 and 7.0 by addition of NaHCO₃ and the mixture stirred for a further 2 hours at 0-5°C, maintaining the pH in the range 6.0 to 7.0, to give a precipitate of azo product which was isolated by filtration and dried.

The above dry azo product (759 parts) and 4-amino-3-carboxybenzene sulphonic acid (227 parts) were stirred in water (4750 parts) at 30-40°C for 4.5 hours, during which the pH was maintained at between 4.0 and 5.0 by addition of NaHCO₃ as required. The resultant mixture was cooled to room temperature and the product isolated by salting out with KCl, the precipitate was dried to give the alkali metal salt of the title compound with absorption maxima at approx. 513nm.

### Example 2

### Preparation of the compound of Formula (6) in which R¹ and R² are H and Y is 2-carboxyphenyl

The method of Example 1 was followed except that in place of 4-amino-3-carboxybenzene sulphonic acid there was used 2-aminobenzoic acid to give the title compound with an absorption maxima at approximately 514nm.

### Example 3

### Preparation of the compound of Formula (6) in which R¹ and R² are H and Y is 4-carboxyphenyl

The method of Example 1 was followed except that in place of 4-amino-3-carboxybenzene sulphonic acid there was used 4-aminobenzoic acid to give the title compound with an absorption maxima at approximately 521nm.

### Example 4

### Preparation of the compound of Formula (6) in which R¹ and R² are H and Y is 3-sulphophenyl

The method of Example 1 was followed except that in place of 4-amino-3-carboxybenzene sulphonic acid there was used 3-aminobenzene sulphonic acid to give the title compound with an absorption maxima at approximately 513nm.

### Example 5

### Preparation of the compound of Formula (6) in which R¹ is OCH₃, R² is H and Y is 2-carboxyphenyl

The method of Example 1 was followed except that in place of 3-beta-sulphatoethylsulphonylaniline there was used 6-methoxy-3-(beta-sulphatoethylsulphonyl)aniline to give the title compound with an absorption maxima at approximately 547nm.

### Example 6

### Preparation of the compound of Formula (6) in which R¹ is H, R² is CH₃ and Y is 2-carboxyphenyl

The method of Example 1 was followed except that in place of 4-amino-3-carboxybenzene sulphonic acid there was used 2-N-methylamino benzoic acid to give the title compound with an absorption maxima at approximately 514nm.

### Example 7

### Preparation of the compound of Formula (6) in which R¹ is H, R² is CH₃ and Y is 4-carboxyphenyl

The method of Example 1 was followed except that in place of 4-amino-3-carboxybenzene sulphonic acid there was used 4-N-methylamino benzoic acid to give the title compound with an absorption maxima at approximately 514nm.

## Claims

1. A compound which, in the free acid form, has the formula: 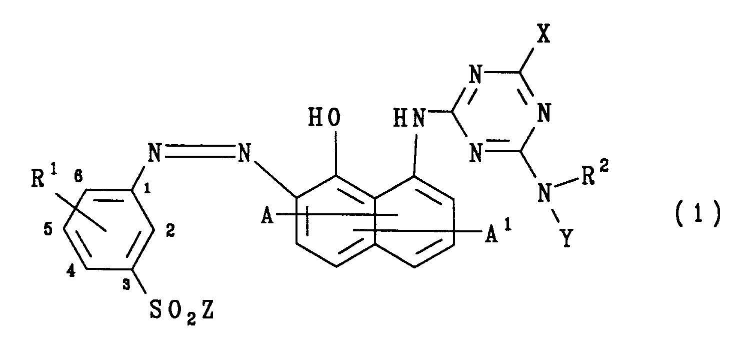 wherein:
R¹ is H, alkyl, alkoxy, CO₂H or SO₃H;
Z is -CH=CH₂ or a group which yields -CH=CH₂ on treatment with aqueous alkali;
A and A¹ are each independently H or SO₃H provided at least one of A and A¹ is SO₃H;
X is Cl or F;
R² is H, alkyl or alkenyl; and
Y is phenyl substituted by one or two groups selected from CO₂H and SO₃H.

2. A compound according to Claim 1 in which Z is -CH=CH₂, -CH₂CH₂SSO₃H or -CH₂CH₂OSO₃H.

3. A compound according to Claim 1 or Claim 2 in which A and A¹ are both SO₃H.

4. A compound according to Claim 3 in which A and A¹ are respectively at the 3- and 5- or 3- and 6- position relative to the OH group connected to the same naphthalene ring system shown in Formula (1).

5. A compound according to any preceding claim in which X is Cl.

6. A compound according to any preceding claim in which R¹ is H, C₁₋₄-alkyl, C₁₋₄-alkoxy, CO₂H or SO₃H.

7. A compound according to any preceding claim in which R¹ is H or methoxy.

8. A compound according to any preceding claim in which R² is H.

9. A compound according to any preceding claim in which Y is 2, 3 or 4-sulphophenyl, 2,4-disulphophenyl, 2,5-disulphophenyl, 3,5-disulphophenyl, 2-carboxyphenyl, 3-carboxyphenyl, 4-carboxyphenyl, 2,3-dicarboxyphenyl, 2,4-dicarboxyphenyl, 2,5-dicarboxyphenyl, 2,6-dicarboxyphenyl, 3,4-dicarboxyphenyl, 3,5-dicarboxyphenyl, 4-carboxy-2-sulphophenyl, 3-carboxy-5-sulphophenyl, 2-carboxy-4-sulphophenyl, or 2-carboxy-5-sulphophenyl.

10. A process for the coloration of materials having -OH and/or -NH- groups by applying thereto a compound defined in any one of Claims 1 to 9.

11. Cellulosic materials having -OH groups, when coloured with a compound defined in any one of Claims 1 to 9.

## Patentansprüche

1. Verbindung, die in der freien Säureform die Formel: 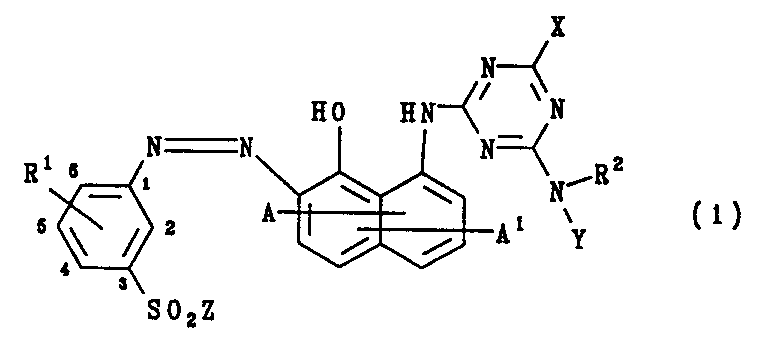 hat, in der:
R¹ H, Alkyl, Alkoxy, CO₂H oder SO₃H ist;
Z -CH=CH₂ oder eine Gruppe, die bei Behandlung mit wäßrigem Alkali -CH=CH₂ liefert, ist;
A und A¹ jeweils unabhängig voneinander H oder SO₃H sind, unter der Voraussetzung, daß mindestens einer der Reste A und A¹ SO₃H ist;
X Cl oder F ist;
R² H, Alkyl oder Alkenyl ist; und
Y Phenyl ist, das durch eine oder zwei Gruppen, die unter CO₂H und SO₃H ausgewählt sind, substituiert ist.

2. Verbindung nach Anspruch 1, in der Z -CH=CH₂, -CH₂CH₂SSO₃H oder -CH₂CH₂OSO₃H ist.

3. Verbindung nach Anspruch 1 oder Anspruch 2, in der A und A¹ beide SO₃H sind.

4. Verbindung nach Anspruch 3, in der A und A¹ in der 3- und 5- oder 3- und 6-Position bezüglich der OH-Gruppe an dasselbe Naphthalin-Ringsystem, das in Formel (1) gezeigt ist, gebunden sind.

5. Verbindung nach irgendeinem der vorangehenden Ansprüche, in der X Cl ist.

6. Verbindung nach einem der vorangehenden Ansprüche, in der R¹, H, C₁₋₄-Alkyl, C₁₋₄-Alkoxy, CO₂H oder SO₃H ist.

7. Verbindung nach irgendeinem der vorangehenden Ansprüche, in der R¹ H oder Methoxy ist.

8. Verbindung nach irgendeinem der vorangehenden Ansprüche, in der R² H ist.

9. Verbindung nach irgendeinem der vorangehenden Ansprüche, in der Y 2,3- oder 4-Sulfophenyl, 2,4-Disulfophenyl, 2,5-Disulfophenyl, 3,5-Disulfophenyl, 2-Carboxyphenyl, 3-Carboxyphenyl, 4-Carboxyphenyl, 2,3-Dicarboxyphenyl, 2,4-Dicarboxyphenyl, 2,5-Dicarboxyphenyl, 2,6-Dicarboxyphenyl, 3,4-Dicarboxyphenyl, 3,5-Dicarboxyphenyl, 4-Carboxy-2-sulfophenyl, 3-Carboxy-5-sulfophenyl, 2,Carboxy-4-sulfophenyl oder 2-Carboxy-5-sulfophenyl ist.

10. Verfahren zur Färbung von Materialien, die -OH- und/oder -NH-Gruppen haben, in dem eine Verbindung, die irgendeinem der Ansprüche 1 bis 9 definiert ist, auf diese Materialien angewendet wird.

11. Cellulose Materialien, die -OH-Gruppen haben, wenn sie mit einer Verbindung, die in irgendeinem der Ansprüche 1 bis 9 definiert ist, gefärbt sind.

## Revendications

1. Composé qui, sous forme d'acide libre, répond à la formule : 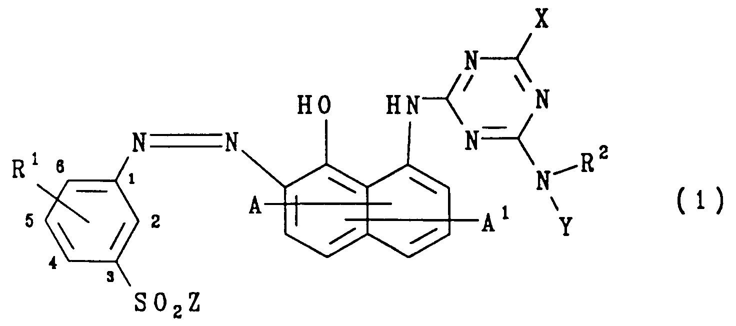 dans laquelle :
R¹ représente H, un groupe alkyle, alkoxy, CO₂H ou SO₃H ;
Z représente un groupe -CH=CH₂ ou un groupe qui donne un groupe -CH=CH₂ par traitement avec une solution aqueuse d'une substance alcaline ;
A et A¹ représentent, chacun, indépendamment, H ou un groupe SO₃H, sous réserve qu'au moins un des groupes A et A¹ représente un groupe SO₃H ;
X représente un groupe Cl ou F ;
R² représente H, un groupe alkyle ou alcényle ; et
Y représente un groupe phényle substitué avec un ou deux groupes choisis entre les groupes CO₂H et SO₃H.

2. Composé suivant la revendication 1, dans lequel Z représente un groupe -CH=CH₂, -CH₂CH₂SSO₃H ou -CH₂CH₂OSO₃H.

3. Composé suivant la revendication 1 ou la revendication 2, dans lequel A et A¹ représentent l'un et l'autre un groupe SO₃H.

4. Composé suivant la revendication 3, dans lequel A et A¹ sont, respectivement, en positions 3 et 5 ou 3 et 6 par rapport au groupe OH connecté au même système cyclique naphtalène représenté dans la formule (1).

5. Composé suivant l'une quelconque des revendications précédentes, dans lequel X représente Cl.

6. Composé suivant l'une quelconque des revendications précédentes, dans lequel R¹ représente H, un groupe alkyle en C₁ à C₄, alkoxy en C₁ à C₄, CO₂H ou SO₃H.

7. Composé suivant l'une quelconque des revendications précédentes, dans lequel R¹ représente H ou un groupe méthoxy.

8. Composé suivant l'une quelconque des revendications précédentes, dans lequel R² représente H.

9. Composé suivant l'une quelconque des revendications précédentes, dans lequel Y représente un groupe 2,3- ou 4-sulfophényle, 2,4-disulfophényle, 2,5-disulfophényle, 3,5-disulfophényle, 2-carboxyphényle, 3-carboxyphényle, 4-carboxyphényle, 2,3-dicarboxyphényle, 2,4-dicarboxyphényle, 2,5-dicarboxyphényle, 2,6-dicarboxyphényle, 3,4-dicarboxyphényle, 3,5-dicarboxyphényle, 4-carboxy-2-sulfophényle, 3-carboxy-5-sulfophényle, 2-carboxy-4-sulfophényle ou 2-carboxy-5-sulfophényle.

10. Procédé pour la coloration de matières ayant des groupes -OH et/ou -NH- par application à ces matières d'un composé répondant à la définition suivant l'une quelconque des revendications 1 à 9.

11. Matières cellulosiques comportant des groupes -OH, ayant été colorées avec un composé répondant à la définition suivant l'une quelconque des revendications 1 à 9.
